Europäisches Patentamt

European Patent Office

Office européen des brevets

(19)

(11) **EP 0 850 629 B1**

(12) **EUROPEAN PATENT SPECIFICATION**

(45) Date of publication and mention
of the grant of the patent:
**22.09.2004 Bulletin 2004/39**

(51) Int Cl.[7]: **A61F 13/15**

(21) Application number: **97122445.6**

(22) Date of filing: **18.12.1997**

(54) **Absorbent member**

Absorbierendes Element

Elément absorbant

(84) Designated Contracting States:
**DE FR GB SE**

(30) Priority: **24.12.1996 JP 34396296**

(43) Date of publication of application:
**01.07.1998 Bulletin 1998/27**

(73) Proprietor: **KAO CORPORATION**
**Chuo-ku, Tokyo (JP)**

(72) Inventors:
• **Minato, Masanori**
**Ichikai-machi, Haga-gun, Tochigi-ken (JP)**

• **Kasai, Takao**
**Ichikai-machi, Haga-gun, Tochigi-ken (JP)**

(74) Representative: **Grünecker, Kinkeldey,**
**Stockmair & Schwanhäusser Anwaltssozietät**
**Maximilianstrasse 58**
**80538 München (DE)**

(56) References cited:
**EP-A- 0 506 336        EP-A- 0 572 569**
**US-A- 4 296 745**

**Description**

The present invention relates to an absorbent member, particularly an absorbent member suitable for use in a thin type absorbent article, such as a disposable diaper and a sanitary napkin, known, for instance, from EP-B-0 572 569.

Absorbent articles, such as disposable diapers, sanitary napkins, and pads for incontinence of urine, comprise an absorbent member which is chiefly arranged in the central part thereof and is to absorb and retain body fluids discharged from the body, e.g., urine or blood, a soft and liquid-permeable top sheet which is arranged on the side coming into contact with the skin, and a liquid-impermeable back sheet which is arranged on the opposite side of the absorbent member. The absorbent member is usually made up of pulp fibers and a water-absorbing polymer. A body fluid, such as urine, that has passed through the top sheet made of nonwoven fabric, etc. is temporarily held in the pulp fibers and then retained in the water-absorbing polymer. Meantime, the water-absorbing polymer swells to increase the volume of the absorbent member. When the amount of the water-absorbing polymer is increased to improve water absorption of the absorbent member, or where the thicknesses of the absorbent article and the absorbent member are to be reduced, the amount of the bulky pulp fibers should be decreased. In this case, the proportion of the pulp in the absorbent member decreases relatively, and entanglement among pulp fibers is reduced. It follows that, while the water-absorbing polymer is dry or when the water-absorbing polymer swells, the absorbent member is liable to be broken or localized under compressive or shearing force as a wearer moves, causing absorption hinderance.

Such absorption hindrance has been avoided by incorporating thermally fusible fibers into pulp fibers so as to produce an adhesive action among the thermally fusible fibers themselves and between the pulp fibers and the thermally fusible fibers to improve shape retention. Proposals concerning incorporation of thermally fusible fibers for improving shape retention are disclosed in Japanese Patent Laid-Open Nos. 92701/88, 318941/88, 260555/88, and 74254/90.

It has also been proposed to mix hydrophilic long fibers, such as rayon, into pulp fibers as disclosed in Japanese Patent Laid-Open No. 98909/94.

However, incorporation of thermally fusible fibers for absorbent member's shape retention has a possibility of causing absorption hindrance because the thermally fusible fibers per se are hydrophobic.

In addition, use of thermally fusible fibers necessitates a heat fusion system in the production line, and the rate of production is restricted because of involvement of the time for heat fusion. Further, the absorbent member tends to become too hard while depending on the kind, amount or fusing temperature of the thermally fusible fibers, which may interfere with swelling of the water-absorbing polymer or deteriorate the texture.

On the other hand, the method comprising mixing hydrophilic long fibers, e.g., rayon, into pulp fibers does not give rise to the above problems. However, the effect in improving shape retention of the absorbent member is lower than that exhibited by the use of thermally fusible fibers. In particular, the effect is insufficient when applied to thin type absorbent articles having a reduced pulp content.

EP-A-572569 discloses an article obtained by impregnating a nonwoven web with a water-soluble binder composition. The article readily dissolves upon contact with an aqueous liquid.

Accordingly, an object of the present invention is to provide thin type absorbent member and absorbent article which can be produced without requiring a heat treatment and yet retain the shape of the absorbent member on absorbing body fluids and are thereby free from absorption hindrances and also which are inhibited from restoring their thickness and have moderate bending stiffness and an excellent texture.

As a result of extensive studies, the inventors of the present invention have found that the above object is accomplished by making an absorbent member of pulp fibers, a water-absorbing polymer and, in addition, fibers or chips different from the pulp fibers (hereinafter simply referred to as different fibers or chips) which manifest adhesiveness among themselves or to the other constituting members on contact with water to thereby stabilize the shape of the absorbent member.

The present invention provides a liquid retentive absorbent member according to claim 1.

The present invention further provides an absorbent article according to claim 8.

Moreover, the present invention provides an absorbent article comprising a liquid-permeable top sheet (2) according to claim 9.

Preferred embodiments of the present invention are set forth in the subclaims.

It seems that, as the absorbent member absorbs a body fluid, the different fibers or chips are moderately plasticized and swollen by the action of the water content of the body fluid to manifest adhesiveness among themselves and to the pulp fibers and water-absorbing polymer, thereby serving to retain the shape of the absorbent member stably on absorbing a body fluid.

Unlike the method of incorporating thermally fusible fibers, the fact that the different fibers or chips used in the present invention are hydrophilic and that they get adhesive almost simultaneously with the water-absorbing polymer's swelling on absorbing a body fluid produces the effect that even thin type absorbent member and absorbent article having a reduced pulp fiber content can retain their shape sufficiently without causing absorption hindrances, such as hindrance to the swelling of the water-absorbing polymer. Further, the absorbent member or absorbent article can be endowed with stable shape retention and inhibited from restoring their thickness while dry by spraying water to the absorbent member to cause the different fibers or chips to manifest adhesiveness previously in the production of the absorbent article.

The absorbent member and absorbent article according to the present invention stably maintain their shape as they absorb body fluids, are free from absorption hindrances, and have an excellent texture.

Fig. 1 schematically illustrates the preparation of a sample for measuring the temperature at which a fiber starts dissolving in water and the percent shrinkage of a fiber.

Fig. 2 schematically illustrates a sample set in a water tank for measuring the temperature at which a fiber starts dissolving in water.

Fig. 3 is a perspective view of a disposable diaper as one embodiment of the absorbent article according to the present invention.

Fig. 4 is a plan view of the disposable diaper of Fig. 3 in its unfolded condition with a part cut away.

Fig. 5 schematically depicts the method of measuring the breaking strength of an absorbent member in the Examples and Comparative Examples.

The absorbent member according to the present invention is a liquid retentive absorbent member mainly comprising pulp fibers and a water-absorbing polymer and further containing fibers or chips different from the pulp fibers (hereinafter sometimes inclusively referred to as different fibers ).

The different fibers manifest adhesiveness among themselves or to the other constituent members, i.e., the pulp fibers and the water-absorbing polymer, on contact with water to thereby stabilize the shape of the absorbent member.

Materials of the fibers useful as "different fibers" include polyvinyl alcohol (PVA), polyvinylpyrrolidone, Poluran, carboxymethyl cellulose (CMC), carboxyethyl cellulose (CEC), hydroxypropyl cellulose, starch, polyethylene glycol (PEG), polyethylene oxide (PEO), polysaccharides, polyvinyl acetate, polyacrylic acid, and some kinds of shape memory materials. Polyvinyl alcohol fibers are particularly preferred. Polyvinyl alcohol modified with maleic acid, itaconic acid, etc. is also useful. These polymeric materials such as polyvinyl alcohol can be incorporated into the absorbent member in the form of not only fibers but chips, such as sheet or ribbon.

The characteristics of the above-enumerated polymeric materials can be controlled so as to perform the above-mentioned function as "different fibers". For example, polyvinyl alcohol fibers can be provided with the function by controlling the molecular weight, degree of saponification, degree of crystallization, and the like. Fibers having the desired function are commercially available under trade names of Kuraray Vinylon and Kremona (both produced by Kuraray Co., Ltd.), Unitika Vinylon (produced by Unitika Ltd.), and Solvlon and Nichibilon (both produced by Nichibi K. K.).

The content of the different fibers is selected appropriately according to the kind of the fibers, preferably ranging from 1 to 50% by weight, particularly 3 to 30% by weight, based on the pulp fibers.

The length of the different fibers is preferably 10 to 100 mm, still preferably 15 to 55 mm, particularly preferably 15 to 30 mm. Fibers shorter than 10 mm have insufficient wet strength. Fibers longer than 100 mm, on the other hand, get entangled among themselves and are difficult to uniformly distribute in an absorbent member, failing to obtain strength. The fineness of the different fibers is preferably 0.09 to 9.09 dtex (0.1 to 10 denier), still preferably 0.45 to 4.54 dtex (0.5 to 5 denier).

When used in the form of chips, the different fibers can take the form of ribbon or any other arbitrary forms having an area of 20 to 500 mm$^2$, preferably 40 to 200 mm$^2$

The different fibers start dissolving in water at a temperature of 50°C or above, particularly 50 to 90°C. The temperature at which the fibers start dissolving in water will hereinafter be referred to as a dissolution initiating temperature. Fibers whose dissolution initiating temperature is lower than 50°C tend to dissolve when the absorbent member absorbs a body fluid.

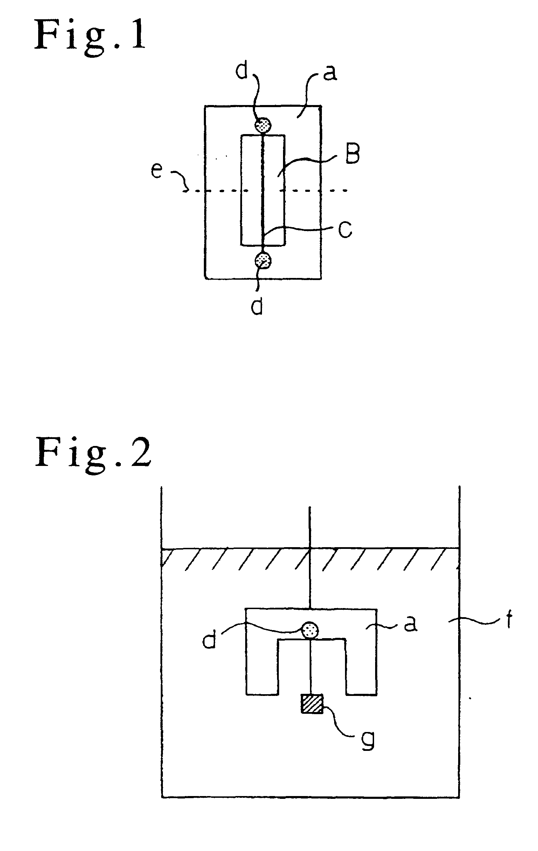

The dissolution initiating temperature of a fiber can be measured as follows. Reference is made to Figs. 1 and 2. As shown in Fig. 1, a rectangular sheet of thin paper a having a width of about 20 mm, a length of about 50 mm, and a thickness of 0.2 mm is cut to make a window b having a width of about 10 mm and a length of 20 mm. A single fiber c is attached across the window b under a slight tension for avoiding slack and fixed at both ends with an adhesive d. The thin paper a is cut across into halves at the middle of the longitudinal direction of the window b along a dotted line e. A weight g is attached to the cut end of the fiber to apply a load of 1/20 cN per denier and immersed in water in a water tank f as shown in Fig. 2. The temperature of water is gradually elevated. The lowest water temperature at which the fiber no more stands the load of the weight g and is cut is taken as a dissolution initiating temperature.

It is also desirable for the different fibers to have a percent shrinkage of 10 to 50%, particularly 20 to 40%, in water at 20°C. If the percent shrinkage of the fibers is less than 10%, sufficient entanglement among themselves and with pulp fibers is not obtained, failing to exert sufficient strength. Fibers whose percent shrinkage exceeds 50% are susceptible to the environmental influences and apt to get lumpy when mixed with pulp, failing to exhibit sufficient strength, either. In such a case, some parts of the absorbent member show strength while the others do not, resulting in breaks in the weaker parts.

The percent shrinkage of the fibers can be determined as follows.

Measurement is made in accordance with "Chemical Fiber Staple Test Method" specified in JIS L 1015-1992. More specifically, reference is made to Fig. 1. A rectangular sheet of thin paper a having a width of about 20 mm, a length of about 50 mm, and a thickness of 0.2 mm is cut to make a window b having a width of about 10 mm and a length of 20 mm. A single fiber c is attached across the window b under a slight tension for avoiding slack and fixed at both ends with an adhesive d. The thin paper a is cut across into halves at the middle of the longitudinal direction of the window b along a dotted line e and immersed in water for 5 minutes. After draining on filter paper, the length of the fiber ($L_2$) is measured under tension enough to take up slack. The percent shrinkage is calculated according to equation (1):

$$\text{Percent Shrinkage (\%)} = [(L_1 - L_2) / L_1] \times 100 \tag{1}$$

wherein $L_1$ is an initial fiber length (mm); and $L_2$ is a fiber length (mm) after immersion.

It is preferable for the different fibers to have a degree of swelling of 30 to 1000%, particularly 40 to 500%, in water at 30°C. If the degree of swelling is less than 30%, the mutual actions exerted among the individual fibers of the different fibers or between the different fibers and the pulp fibers, such as adhesion or entanglement, tend to be insufficient for obtaining sufficient strength. Fibers whose degree of swelling exceeds 1000% reduce their own strength due to swelling, also tending to have insufficient strength.

The degree of swelling of the fibers can be measured as follows. Fibers precisely weighing 10 g are packed into a bag of nylon mesh and immersed in water at 30°C for 30 minutes. The bag and the contents were dehydrated by centrifugation at 800 rpm, and the weight of the swollen fibers was measured. The degree of swelling is calculated from the following equation:

$$\text{Degree of Swelling (g)} =$$

$$[(\text{weight after swelling (g)} - \text{initial weight (g)})/\text{initial weight (g)}] \times 100$$

Any pulp fibers which have conventionally been in use can be used in the present invention with no particular limitation. Taking the steps of grinding, building up, compressing, etc. into consideration, the pulp fibers preferably have an average fiber length of 0.8 to 3 mm.

Any water-absorbing polymer which has conventionally been in use can be used in the present invention with no particular limitation. In particular, superabsorbent polymers are preferably used. The superabsorbent polymers preferably have a rate of liquid absorption of 20 wt/wt or more.

It is preferable that the superabsorbent polymer has a liquid passage time of not more than 20 seconds as measured by charging 0.5 g of the superabsorbent polymer in a cylinder having a cross-sectional area of 4.91 cm$^2$ (inner diameter: 25 mm$\phi$) together with physiological saline to swell the superabsorbent polymer with saline to saturation and, after the swollen superabsorbent polymer has sunk, measuring the time required for 50 ml of physiological saline to pass through the superabsorbent polymer.

The "liquid passage time" is a measure of occurrence of gel blocking of a superabsorbent polymer. If the liquid passage time is longer than 20 seconds, the superabsorbent polymer, being used in a proportion of 45% by weight or more based on the total weight of the absorbent member, causes gel blocking and is hindered from exercising its absorption performance. The shorter the liquid passage time, the better, but the practice being taken into consideration, a preferred range is 2 to 20 seconds, particularly 2 to 15 seconds.

Examples of suitable water-absorbing polymers are powders of sodium polyacrylate, an acrylic acid-vinyl alcohol copolymer, a crosslinked sodium polyacrylate, a starch-acrylic acid graft polymer, an isobutylene-maleic anhydride copolymer or a saponification product thereof, potassium polyacrylate, and cesium polyacrylate.

The water-absorbing polymer is preferably used in an amount of 100 to 500% by weight, particularly 120 to 300% by weight, based on the pulp fibers. As long as the water-absorbing polymer is used in an amount within this range, the absorbent member is capable of sufficiently absorbing body fluids and sufficiently retains its shape.

The different fibers or chips can be mixed with the water-absorbing polymer and pulp fibers uniformly, or the different fibers or chips can be mixed with the pulp fibers, and the water-absorbing polymer is separately scattered. Mixing of the different fibers with the pulp fibers (and the water-absorbing polymer) does not need to be completely uniform. For example, the different fibers may exist in groups of a given amount, the different fibers may be shaped into nonwoven sheeting on which the pulp fibers or water-absorbing polymer is built up or which can be sandwiched in between a layer of the pulp fibers and a layer of the water-absorbing polymer, or the different fibers may be shaped into nonwoven sheeting in which a mixture of the pulp fibers and the water-absorbing polymer can be wrapped.

The distribution of the pulp fibers or water-absorbing polymer in the absorbent member can be designed appropriately according to the purpose. For instance, the water-absorbing polymer is scattered in a limited width to be localized in the central portion in the width direction, or it is heavily concentrated in the urination area to make that area thicker. The water-absorbing polymer is distributed in the front portion and the rear portion at a ratio of 100/0 (front/rear) to 30/70, preferably 80/20 to 40/60.

The absorbent member according to the present invention is particularly advantageous for use as a thin type absorbent member. Accordingly, the absorbent member of the present invention has a thickness of not more than 10 mm, preferably 5 mm or less, still preferably 3 mm or less. It is desirable for the absorbent member having a thickness of 5 mm or less to have a breaking strength of not less than 100 cN/70 mm. If the breaking strength is less than 100 cN/70 mm, the absorbent member tends to break appreciably while worn, causing leaks. It is also preferable for the absorbent member to have a Taber stiffness, specified in JIS P 1825, of less than 20 g in average. It the absorbent member has an average Taber stiffness of 20 g or more, the absorbent article such as a disposable diaper is considerably hard.

The thin type absorbent member has a basis weight of 50 to 500 $g/m^2$, preferably 50 to 300 $g/m^2$. An absorbent member having a basis weight of less than 50 $g/m^2$ is short of absorption capacity. If the basis weight exceeds 500 $g/m^2$, the absorbent member is too thick for a fit while worn or unfavorably bulky to carry.

The absorbent article according to the present invention comprises a liquid-permeable top sheet, a liquid-impermeable back sheet, and a liquid retentive absorbent layer interposed therebetween, wherein the absorbent layer is the above-described absorbent member.

Where it is desirable to use a thin type absorbent layer in the absorbent article, the absorbent layer is made up of two or more layers, at least one of which is a liquid diffusing layer made of a fibrous base material having a high void, and the above-described absorbent member is used as a layer below the liquid diffusing layer.

The liquid diffusing layer made of a fibrous base material having a high void is, for example, a built-up layer composed of a plurality of sheets of nonwoven fabric having a high void. This layer functions as a layer for temporarily holding a body fluid, a liquid diffusing layer, or a separator from the skin, thereby enhancing the absorption performance of the absorbent member, i.e., rapidness in absorption and resistance against back-flow of body fluids.

The fibrous base material having a high void includes cellulose fibers, modified cellulose fibers, synthetic fibers, and mixtures of two or more thereof.

The cellulose fibers include natural fibers, such as wood pulp and cotton, and cellulosic chemical fibers, such as viscous rayon and acetate.

The synthetic fibers include polyethylene fibers, polypropylene fibers, polyester fibers, polyamide fibers, polyacrylonitrile fibers, polyvinyl alcohol fibers, conjugated fibers composed of two or more of these synthetic fibers in the form of core/sheath, etc., and mixed fibers comprising two or more of these synthetic fibers. Of the fibrous base materials, the synthetic fibers are preferably used after having their surface rendered hydrophilic by a corona treatment, a plasma treatment, or a treatment with a chemical for making the surface hydrophilic, such as a surface active agent.

The layer comprising a fibrous base material having a high void is preferably a built-up layer composed of a plurality of sheets of nonwoven fabric fabricated of synthetic fibers having a high void. The nonwoven fabric to be used preferably has a basis weight of 15 to 70 $g/m^2$, still preferably 20 to 60 $g/m^2$. Preferred fibers constituting the nonwoven fabric include polyethylene fibers, polypropylene fibers, PET fibers or rayon fibers having a fiber diameter of 2.72 to 9.09 dtex (3 to 10 denier) and a fiber length of 38 to 75 mm. A dry-processed pulp sheet mainly comprising pulp fibers and a binder component may be used. Further, porous films and foamed sheets (urethane or olefin foamed sheets) are also useful.

The top sheet of the absorbent article of the present invention is not particularly limited as far as it has sufficient permeability to liquid. For example, woven fabric, nonwoven fabric or a porous sheet can be used. Materials of the top sheet include rayon, cotton, polyester, polyethylene, and polypropylene. Any liquid-impermeable sheet that has conventionally been used in absorbent articles can be used as a back sheet with no particular limitation. A steam-permeable sheet impermeable to liquid and permeable to steam which is obtained by stretching a filler-containing thermoplastic resin sheet and an air-permeable film made of certain kinds of urethane or polyester are preferably used. A composite material composed of such a sheet and nonwoven fabric is also useful.

The process for producing the absorbent article of the present invention preferably includes a step of adding water to the absorbent member. Previous addition of water is effective for the absorbent article to stably retain its shape while dry and on absorption of a body fluid. The amount of water to be added is preferably less than 30 parts by weight per 100 parts by weight of the absorbent member. Where water is added to the absorbent member, the water activity value of the absorbent member in the equilibrium state is preferably less than 0.6. If the water activity value exceeds 0.6, mold can generate, and the absorbent member is so hard that the texture is deteriorated. Other steps for the production of the absorbent article of the present invention are carried out in a manner well known in the art.

The absorbent article of the present invention is suitably applied to disposable diapers and, in addition, other articles, such as sanitary napkins, pads for incontinence of urine, nursing breast pads, mats for bed-wetting, sheets for the bed ridden, sheets for pets and the like.

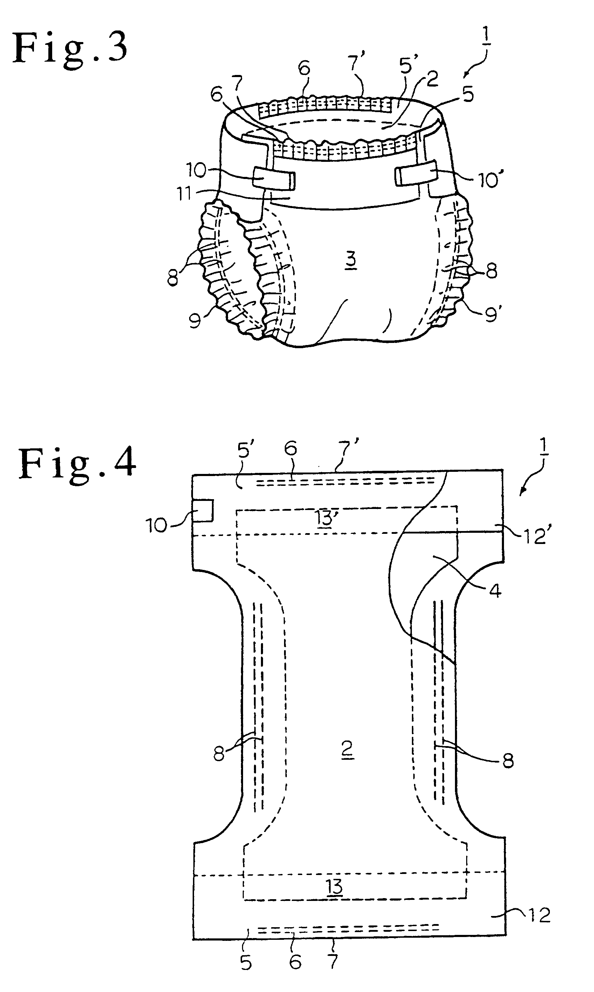

Preferred embodiments of the absorbent article according to an aspect of the invention will be described taking, for instance, a disposable diaper by referring to the accompanying drawings. Fig. 3 is a perspective view of a disposable diaper as a first embodiment of the absorbent article of the first aspect of the invention. Fig. 4 is a plan of the disposable diaper shown in Fig. 3 in its laid-flat state, with a part thereof cutaway.

The disposable diaper 1 of the embodiment shown in Figs. 3 and 4 comprises a liquid permeable topsheet 2, a liquid impermeable backsheet 3, and a liquid-retentive absorbent member 4 that is interposed between the topsheet 2 and the backsheet 3. It has a front waist portion 5 and a rear waist portion 5' which, when the diaper is worn, correspond to the front body portion and the rear body portion of a wearer, respectively.

The sides of the rear waist portion 5' of the disposable diaper 1 are provided with a pair of fastening tapes 10 and 10' by which the front waist portion 5 and the rear waist portion 5' are fastened together when the diaper is worn. On the surface of the backsheet 3 in the front waist portion 5 is provided a so-called landing tape 11 to which the fastening tapes 10 and 10' are to be stuck.

The absorbent member 4 has a portion corresponding to the crotch portion of the diaper to have a sandglass shape. Elastic members 6 for the waist and elastic members 8 for legs are provided around the absorbent member 4, i.e., at the front and rear waist portions 5 and 5' and both leg opening portions. These elastic members are fixed in their extended state between the topsheet 2 and the backsheet 3 so that they contract in their free state to form waist gathers 7 and 7' and leg gathers 9 and 9' at the waist opening portion and the leg opening portions thereby giving a good fit on the wearer's body as illustrated in Fig. 3.

In the disposable diaper 1, the topsheet 2 is a liquid permeable sheet through which body waste passes into the absorbent member 4 and is preferably made of a material feeling like underwear. Such a liquid permeable sheet preferably includes woven fabric, nonwoven fabric, and perforated film. A topsheet 2 with its peripheral portion rendered water-repellent to prevent leakage of urine, etc. due to oozing from the periphery can also be used preferably. Treatment for making the sheet water repellent can by carried out by coating the peripheral portion of the topsheet 2 with a hydrophobic compound such as silicone oil or paraffin wax or coating the entire surface of the topsheet 2 with a hydrophilic compound such as an alkyl phosphate and then washing the peripheral portion with warm water.

The backsheet 3 is a moisture permeable and liquid impermeable sheet which is impermeable to liquid but permeable to water vapor and is preferably made of a material having a feeling like underwear. Such a liquid impermeable sheet can be obtainable by stretching a thermoplastic resin containing a filler. In particular, the liquid impermeable sheet is preferably made of a microporous sheet having moisture permeability and a composite sheet comprising such a microporous sheet and nonwoven fabric. The details of the microporous sheet which is preferably used in the present invention will be described later.

The elastic members 6 for the waist gathers 7 and 7' and the elastic members 8 for the leg gathers 9 and 9' are preferably rubber strings, rubber braid, elastic films or foamed films of polyurethane.

The present invention will now be illustrated in greater detail by way of Examples.

(1) Preparation of Disposable Diapers

<u>EXAMPLE 1</u>

On tissue having a basis weight of 15 g/m$^2$ was built up a uniform mixture consisting of 60 parts of fibillated pulp fibers, 5 parts of polyvinyl alcohol (PVA) fibers having a dissolution initiating temperature of 60°C (degree of polymerization: 1700; completely saponified; 1.81 dtex (2 denier); 15-mm cut fibers), and 100 parts of a superabsorbent polymer to a basis weight of 250 g/m$^2$ and enveloped in the tissue to obtain an absorbent member having a thickness of 2 mm. Nonwoven fabric made up of polyethylene fibers and having a basis weight of 25 g/m$^2$ and a polyethylene sheet were put on the face and the back of the absorbent member as a top sheet and a back sheet, respectively, to obtain a disposable diaper.

<u>EXAMPLE 2</u>

Disposable diapers were prepared in the same manner as in Example 1, except for replacing the PVA fibers used in Example 1 with 10 parts of other PVA fibers (degree of polymerization: 2400; completely saponified; 1.81 dtex (2 denier); 15-mm cut fibers) having a dissolution initiating temperature of 90°C.

EXAMPLE 3

Disposable diapers were prepared in the same manner as in Example 1, except for using 20 parts of the PVA fibers used in Example 1.

EXAMPLE 4

Disposable diapers were prepared in the same manner as in Example 1, except for replacing the PVA fibers used in Example 1 with 5 parts of other PVA fibers (degree of polymerization: 1700; completely saponified; 1.36 dtex (1.5 denier); 20-mm cut fibers) having a dissolution initiating temperature of 80°C.

COMPARATIVE EXAMPLE 1

Disposable diapers were prepared in the same manner as in Example 1, except for replacing the PVA fibers used in Example 1 (dissolution initiating temperature: 60°C) with water-insoluble rayon fibers (1.36 dtex (1.5 denier); 15-mm cut fibers).

COMPARATIVE EXAMPLE 2

Disposable diapers were prepared in the same manner as in Example 2, except for replacing the PVA fibers used in Example 2 (dissolution initiating temperature: 90°C) with other PVA fibers (1.81 dtex (2 denier); 15-mm cut fibers) having a dissolution initiating temperature of 5°C or less.

COMPARATIVE EXAMPLE 3

Disposable diapers were prepared in the same manner as in Example 1, except that the PVA fibers used in Example 1 (dissolution initiating temperature: 60°C) were replaced with polyethylene (PE) fibers (1.81 dtex (2 denier); melting point: 100°C; 10-mm cut fibers), and the resulting absorbent member was subjected to heat treatment with a hot embossing roll having a surface temperature of 140°C.

(2) Evaluation of Disposable Diapers

Two hundred disposable diapers prepared in each of Examples 1 to 4 and Comparative Examples 1 to 2 were tested in actual use on 20 wearers to obtain the ratio of diapers that had urine leakage to the total diapers (200) (designated ratio A). Further, the diapers after use were collected, and the absorbent members were observed to obtain the ratio of diapers the absorbent member of which had broken to the total diapers (designated ratio B). Furthermore, the breaking strength of the absorbent member was measured in accordance with the following method. The results obtained are shown in Table 1 below.

Measurement of Breaking Strength:

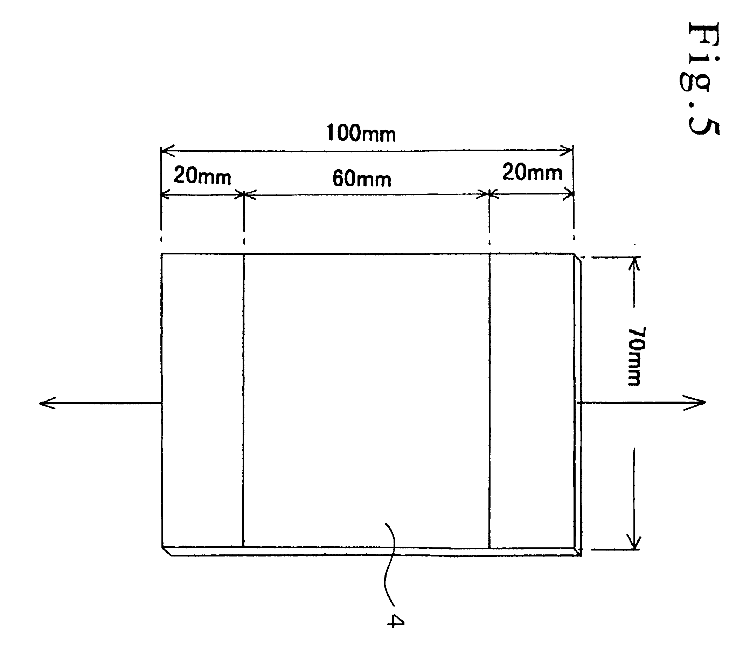

As shown in Fig. 5, a 100 mm long (MD) and 70 mm wide (CD) sample was cut out of the absorbent member, and 0.5 g/cm$^2$ of physiological saline was absorbed therein. After complete absorption by the water-absorbing polymer, Both 20-mm wide ends of the sample were pinched with a pair of grips, and a breaking strength was measured with Tensilon (manufactured by TOYO-WALDWIN) at a grip separation rate of 300 m/min. Measurements were made on 5 points, and the results were averaged.

TABLE 1

|  | Fiber Added | Dissolution Initiating Temp. (°C) | Ratio A (%) | Ratio B (%) | Breaking Strength (cN/70 mm) |
|---|---|---|---|---|---|
| Example 1 | PVA | 60 | 1.0 | 2.0 | 200 |
| Example 2 | PVA | 90 | 0.5 | 1.0 | 340 |
| Example 3 | PVA | 60 | 1.0 | 1.5 | 380 |

TABLE 1   (continued)

|  | Fiber Added | Dissolution Initiating Temp. (°C) | Ratio A (%) | Ratio B (%) | Breaking Strength (cN/70 mm) |
|---|---|---|---|---|---|
| Example 4 | PVA | 80 | 0.5 | 0.5 | 400 |
| Compara. Example 1 | rayon | insoluble | 4.5 | 23.5 | 80 |
| Compara. Example 2 | PVA | <5 | 7.5 | 35.0 | 50 |
| Compara. Example 3 | PE | insoluble | 8.5 | 1.5 | 400 |

The following conclusions are derived from the results of the Examples and Comparative Examples.

(a) The disposable diapers having an absorbent member comprising a mixture of pulp fibers and PVA fibers as different fibers (Examples 1 to 4) show few urine leaks. The absorbent members used therein have a high breaking strength and undergo few breaks.

(b) The disposable diapers in which rayon (Comparative Example 1) or PVA fibers (Comparative Example 2), both of which have no function as specified in the present invention are used in the absorbent member show a high ratio of urine leakage. The absorbent members used therein have a poor breaking strength and show high ratios of breaks.

(c) The disposable diaper in which the absorbent member containing PE fibers which do not perform the function specified in the present invention is subjected to heat fusion treatment (Comparative Example 3) show a high ratio of urine leakage, though the absorbent member thereof has a satisfactory breaking strength and a low ratio of breaks.

## Claims

1.  A liquid retentive absorbent member mainly comprising pulp fibers and a water-absorbing polymer and further containing fibers or chips different from the pulp fibers, wherein said fibers or chips different from the pulp fibers, on contact with water, get adhesive almost simultaneously with the water-absorbing polymer's swelling to stabilize the shape of the absorbent member, wherein said fibers or chips different from the pulp fibers starting dissolving in water at a temperature of 50 °C or above, and wherein said fibers or chips different from the pulp fibers are present in an amount of from 1 to 50% by weight based on the pulp fibers.

2.  The absorbent member of claim 1, which has a breaking strength of not less than 100 cN/70 mm with its thickness being 5 mm or less.

3.  The absorbent member of claim 1 or 2, which has a Taber stiffness of less than 20 g in average, measured according to JIS P 1825.

4.  The absorbent member of claim 1, 2 or 3, wherein said fibers or chips different from the pulp fibers are made from polyvinyl alcohol or modified polyvinyl alcohol.

5.  The absorbent member of any one of claims 1 to 4, wherein said water-absorbing polymer is present in an amount of from 100 to 500% by weight based on the pulp fibers.

6.  The absorbent member of any one of claims 1 to 5, wherein said fibers different from the pulp fibers have a fiber length of from 10 to 100 mm.

7.  The absorbent member of any one of claims 1 to 6, wherein said fibers different from the pulp fibers have a fineness of from 0.09 to 9.09 dtex (0.1 to 10 denier).

8.  An absorbent article comprising a liquid-permable top sheet (2), a liquid-impermeable back sheet (3), and a liquid retentive absorbent layer interposed therebetween, wherein said absorbent layer is the absorbent member of any one of claims 1 to 7.

9. An absorbent article comprising a liquid-permeable top sheet (2), a liquid-impermeable back sheet (3), and a liquid retentive absorbent layer interposed therebetween, wherein said absorbent layer is composed of two or more layers, at least one of which is a liquid diffusing layer made of a fibrous base material having a high void, and the absorbent member of any one of claims 1 to 7 is used as a layer below said liquid diffusing layer.


**Patentansprüche**

1. Flüssigkeit zurückhaltendes absorptionsfähiges Element, welches im Wesentlichen Zellstoff-Fasern und ein Wasser absorbierendes Polymer umfasst, und welches darüber hinaus Fasern oder Späne enthält, die von den Zellstoff-Fasern verschieden sind, wobei besagte von den Zellstoff-Fasern verschiedene Fasern oder Späne bei Kontakt mit Wasser annähernd zur gleichen Zeit, zu der das Wasser absorbierende Polymer aufquillt, adhäsiv werden, um die Form des absorptionsfähigen Elementes zu stabilisieren, wobei besagte von den Zellstoff-Fasern verschiedene Fasern oder Späne bei einer Temperatur von 50 °C oder darüber beginnen, sich in Wasser aufzulösen, und wobei besagte von den Zellstoff-Fasern verschiedene Fasern oder Späne in einer Menge von 1 bis 50 Gewichts% bezogen auf die Zellstoff-Fasern vorliegen.

2. Absorptionsfähiges Element nach Anspruch 1, welches eine Reißfestigkeit von nicht weniger als 100 cN / 70 mm aufweist, bei einer Dicke von 5 mm oder weniger.

3. Absorptionsfähiges Element nach Anspruch 1 oder 2, welches eine TABER-Steifigkeit von im Durchschnitt weniger als 20 g aufweist, gemessen gemäß JIS P 1825.

4. Absorptionsfähiges Element nach Anspruch 1, 2 oder 3, wobei besagte von den Zellstoff-Fasern verschiedene Fasern oder Späne aus Polyvinylalkohol oder modifiziertem Polyvinylalkohol hergestellt sind.

5. Absorptionsfähiges Element nach irgendeinem der Ansprüche 1 bis 4, wobei besagtes Wasser absorbierendes Polymer in einer Menge von 100 bis 500 Gewichts% bezogen auf die Zellstoff-Fasern vorliegt.

6. Absorptionsfähiges Element nach irgendeinem der Ansprüche 1 bis 5, wobei besagte von den Zellstoff-Fasern verschiedene Fasern eine Faserlänge von 10 bis 100 mm aufweisen.

7. Absorptionsfähiges Element nach irgendeinem der Ansprüche 1 bis 6, wobei besagte von den Zellstoff-Fasern verschiedene Fasern eine Feinheit von 0,09 bis 9,09 dtex (0,1 bis 10 Denier) besitzen.

8. Absorptionsfähiger Gegenstand, umfassend eine Flüssigkeits-durchlässige Oberlage (2), eine Flüssigkeits-undurchlässige rückseitige Lage (3) und eine dazwischen eingebrachte Flüssigkeit zurückhaltende, absorptionsfähige Schicht, wobei besagte absorptionsfähige Schicht das absorptionsfähige Element nach irgendeinem der Ansprüche 1 bis 7 darstellt.

9. Absorptionsfähiger Gegenstand, umfassend eine Flüssigkeits-durchlässige Oberlage (2), eine Flüssigkeits-undurchlässige rückseitige Lage (3) und eine dazwischen eingebrachte Flüssigkeit zurückhaltende, absorptionsfähige Schicht, wobei besagte absorptionsfähige Schicht aus zwei oder mehr Schichten zusammengesetzt ist, wobei mindestens eine dieser Schichten eine eine Flüssigkeit verbreitende Schicht darstellt, welche aus einem faserigen Basismaterial, das in einem hohen Maße Hohlräume aufweist, hergestellt ist, und wobei das absorptionsfähige Element nach irgendeinem der Ansprüche 1 bis 7 als eine Schicht verwendet wird, die sich unterhalb besagter, eine Flüssigkeit verbreitender Schicht befindet.


**Revendications**

1. Elément absorbant de rétention de liquide comprenant principalement des fibres de pâte et un polymère absorbant l'eau et contenant de plus des fibres ou copeaux différents des fibres de pâte, dans lequel lesdites fibres ou lesdits copeaux différents des fibres de pâte, lors d'un contact avec l'eau, deviennent adhésifs pratiquement simultanément au gonflement du polymère absorbant l'eau pour stabiliser la forme de l'élément absorbant, dans lequel lesdites fibres ou lesdits copeaux différents des fibres de pâte commencent à se dissoudre dans l'eau à une température de 50°C ou plus, et dans lequel lesdites fibres ou lesdits copeaux différents des fibres de pâte sont présents selon une quantité de 1 à 50 % en poids sur la base des fibres de pâte.

2.  Elément absorbant selon la revendication 1, qui a une résistance à la rupture qui n'est pas inférieure à 100 cN/70 mm avec son épaisseur qui est de 5 mm ou moins.

3.  Elément absorbant selon la revendication 1 ou 2, qui a une rigidité Taber inférieure à 20 g en moyenne, mesurée conformément à la norme JIS P 1825.

4.  Elément absorbant selon la revendication 1, 2 ou 3, dans lequel lesdites fibres ou lesdits copeaux différents des fibres de pâte sont constitués d'un alcool polyvinylique ou d'un alcool polyvinylique modifié.

5.  Elément absorbant selon l'une quelconque des revendications 1 à 4, dans lequel ledit polymère absorbant l'eau est présent selon une quantité allant de 100 à 500 % en poids sur la base des fibres de pâte.

6.  Elément absorbant selon l'une quelconque des revendications 1 à 5, dans lequel lesdites fibres différentes des fibres de pâte ont une longueur de fibre allant de 10 à 100 mm.

7.  Elément absorbant selon l'une quelconque des revendications 1 à 6, dans lequel lesdites fibres différentes des fibres de pâte ont une finesse allant de 0,09 à 9,09 dtex (0,1 à 10 deniers).

8.  Article absorbant comportant une feuille supérieure perméable aux liquides (2), une feuille arrière imperméable aux liquides (3), et une couche absorbante de rétention de liquide interposée entre celles-ci, dans lequel ladite couche absorbante est l'élément absorbant selon l'une quelconque des revendications 1 à 7.

9.  Article absorbant comportant une feuille supérieure perméable aux liquides (2), une feuille arrière imperméable aux liquides (3), et une couche absorbante de rétention de liquide interposée entre celles-ci, dans lequel ladite couche absorbante est constituée de deux ou plus de deux couches, dont au moins une est une couche de diffusion de liquide constituée d'un matériau à base de fibres présentant un grand vide, et l'élément absorbant selon l'une quelconque des revendications 1 à 7 est utilisé en tant que couche située au-dessous de ladite couche de diffusion de liquide.

# Fig.1

# Fig.2

# Fig.3

# Fig.4

# Fig.5